(19) Europäisches Patentamt
European Patent Office
Office européen des brevets

(11) Numéro de publication: **0 043 757**
**B1**

(12) # FASCICULE DE BREVET EUROPEEN

(45) Date de publication du fascicule du brevet: **09.11.83**

(51) Int. Cl.³: **C 07 C 29/62**, C 07 C 31/34

(21) Numéro de dépôt: **81401032.8**

(22) Date de dépôt: **26.06.81**

(54) **Procédé de préparation de iodo alcools à chaîne polyfluoroalkyle.**

(30) Priorité: **08.07.80 FR 8015122**

(43) Date de publication de la demande: **13.01.82 Bulletin 82/2**

(45) Mention de la délivrance du brevet: **09.11.83 Bulletin 83/45**

(84) Etats contractants désignés: **BE CH DE FR GB IT LI NL SE**

(56) Documents cités:
**FR - A - 2 103 459**
**FR - A - 2 359 133**
**GB - A - 1 017 978**

(73) Titulaire: **P C U K PRODUITS CHIMIQUES UGINE KUHLMANN**, *Service Propriété Industrielle Tour* **Manhattan, F-92087 Paris La Défense 2 Cédex 21 (FR)**

(72) Inventeur: **Commeyras, Ausguste André Aimé, 6 Impasse des Ecoles, F-34960 Clapiers (FR)**
Inventeur: **Sagnes, René Félix Alphonse, 1560 Rue Pioch du Boutonnet, F-34100 Montpellier (FR)**
Inventeur: **Guery, Claudine Marcelle, Les Lierres - 4, rue Paul Verlaine, F-34100 Montpellier (FR)**

(74) Mandataire: **Bernard, Nicole et al, PCUK Produits** *Chimiques Ugine Kuhlmann Service Propriété* **Industrielle Tour Manhattan - Cedex 21, F-92087 Paris la Défense 2 (FR)**

**EP 0 043 757 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Procédé de préparation de iodo-alcools à chaîne polyfluoroalkyle

Le procédé de l'invention concerne la préparation d'iodo-alcools à chaîne perfluoroalkyle.

Ce procédé consiste à additionner un iodure de perfluoroalkyle $CF_3(CF_2)_n I$ que nous désignerons sous le terme générique $R_FI$ à un alcool éthylénique.

Il existe divers procédés permettant d'additionner les iodures $R_FI$ à des composés éthyléniques. Cependant ces procédés ne donnent que des rendements médiocres dans le cas des alcools éthyléniques et notamment dans le cas de l'alcool allylique.

Cette addition peut s'effectuer par exemple par voie radicalaire initiant la réaction par une élévation de température (R. N. Haszeldine, J. Chem. Soc., 1953, p. 1199, USP 3 016 406 et 3 016 407), par irradiation par les rayons UV (article de R. N. Haszeldine déjà cité, J. D. Park, J. Org. Chem., 26, 1961, p. 2086, et D. Cantacuzene, J. Chem. Soc. Perkin I, 1977, p. 1365), par l'intermédiaire des dérivés azoïques (N. O. Brace, J. Org. Chem., 27, 1962, p. 3027, et USP 3 083 224, 3 145 222 et 3 257 407).

Il est également possible de catalyser l'addition par le système d'Assher (J. Chem. Soc., 1961, p. 2261), le catalyseur étant un mélange de sels cuivreux et cuivriques et d'amines. Ce procédé a été étendu à des molécules fluorées par D. J. Burton (Tetrahedron Letters, 1966, p. 5163) et N. O. Brace (J. Org. Chem., 44 [1979], p. 212). Un procédé voisin est décrit dans le brevet français N° 2 103 459, qui utilise comme catalyseur un mélange d'au moins une amine avec au moins un sel d'un métal des groupes Ia à IVa ou Ib à VIIIb du système périodique, pris éventuellement sous forme d'un complexe d'amine avec un sel métallique.

Le brevet français N° 2 359 133 décrit également l'addition d'un dérivé au moins gem-dihalogéné sur des alcools du type allylique en présence de sels d'éléments de transition, éventuellement complexés avec une amine.

Le brevet britannique N° 1 017 978 enseigne que l'addition du tétrachlorure de carbone, du tétrabromure de carbone ou d'un alkyltrihalogénométhane à un alcool éthylénique est catalysée par un halogénure de métal possédant deux degrés d'oxydation qui diffèrent par un électron, comme les halogénures de fer, de cuivre, de cobalt, de chrome, de nickel, de mercure et métaux similaires.

Si certains de ces systèmes catalytiques permettent d'additionner un iodo-perfluoroalcane à un composé éthylénique, ils présentent l'inconvénient de nécessiter le plus souvent des conditions sévères de réaction et de n'aboutir qu'à des rendements médiocres et très variables suivant la nature de l'initiateur ou du catalyseur et celle de l'oléfine utilisée. En particulier il n'existe pas de système capable de provoquer l'addition quantitative d'un iodo-perfluoroalcane sur les alcools éthyléniques dans des conditions douces de réaction. C'est ainsi que l'addition photochimique suivant J. D. Park, ou le procédé décrit dans le brevet français N° 2 103 459 donnent un taux de transformation de 50 – 55% seulement avec l'alcool allylique.

La demanderesse a mis au point un procédé qui utilise comme catalyseurs certains sels des couples $Hg^{++}/Hg^{+}$ ou $Mn^{3+}/Mn^{++}$. Ces catalyseurs permettent d'opérer à une température proche de la température ambiante, l'addition pratiquement quantitative de $R_FI$ aux alcools éthyléniques et en particulier à l'alcool allylique. La réaction est conduite de préférence en présence d'un excès d'alcool qui peut jouer le rôle de solvant avec éventuellement adjonction d'un autre solvant tel que le DMF par exemple, ce qui augmente la vitesse de réaction. Celle-ci est en général très rapide et il est en particulier possible d'obtenir des transformations quantitatives dans le cas de l'alcool allylique avec des temps de réaction de l'ordre de 2 heures.

Les divers paramètres: excès d'alcool, rapport $Hg^{++}/Hg^{+}$, rapport $Hg/R_FI$, température, ne sont pas critiques. Toutefois, pour obtenir des rendements quantitatifs avec des durées de réaction aussi courtes que possible, il est préférable d'employer un excès d'alcool et un rapport $Hg^{+}/Hg^{++}$ inférieur à 1. De même, la vitesse de réaction augmente avec la température, mais en pratique il est inutile de dépasser des températures de l'ordre de 35 à 40° C.

La longueur de la chaîne perfluoroalkyle n'a que peu d'influence sur le rendement et la vitesse de réaction. Celle-ci doit être arrêtée dès que le $R_FI$ est entièrement consommé, sinon l'iodhydrine formée risque de se dégrader.

Les couples les plus efficaces sont les iodures mercureux/mercurique, le couple acétate mercureux/iodure mercurique, et le couple acétylacétonates de $Mn^{++}/Mn^{3+}$.

Avec l'alcool allylique et les alcools non saturés $\alpha\omega$ on obtient un seul isomère suivant la réaction:

$$R_FI + CH_2{=}CH-CH_2OH \rightarrow R_F-CH_2-CHI-CH_2OH$$

En revanche, avec les alcools ayant une double liaison interne ou une substitution sur le carbone adjacent, qu'ils soient primaires ou secondaires, on obtient un mélange d'isomères.

Tous ces produits sont des intermédiaires et peuvent être utilisés pour la fabrication de tensio-actifs fluorés et pour l'obtention de dérivés hydro et oléophobes utiles en particulier pour le traitement des textiles, cuir, papier, etc.

Les exemples suivants illustrent l'invention sans toutefois la limiter.

Exemple 1

Dans un réacteur de 250 ml avec agitateur magnétique on met 0,1 mole (5,8 g) d'alcool allylique, 5 ml de DMF, 1,5 · $10^{-3}$ moles (0,98 g) de $Hg_2I_2$, 3 · $10^{-3}$ moles (1,36 g) de $HgI_2$ et 20 · $10^{-3}$ moles (8,92 g) de $C_6F_{13}I$.

On porte à 35° C. Au bout de 1 h 30, tout le $R_FI$ est consommé et transformé en produit d'addition $C_6F_{13}-CH_2-CHI-CH_2OH$. Le rendement vérifié par C.P.V., RMN ($H^1$ et $F^{19}$) est quantitatif.

Il est possible de séparer le produit de la réaction en versant le mélange réactionnel dans 250 ml d'eau. La couche dense est décantée et extraite avec du $CCl_4$. Après séchage, traitement au noir animal et évaporation du $CCl_4$, on obtient 17 · $10^{-3}$ moles (8,57 g) d'iodhydrine (rendement 85%) qui se présente sous la forme d'un solide blanc sublimable de point de fusion 49° C.

Exemples 2 à 5

On conduit diverses opérations en changeant les paramètres de la réaction et la longueur de la chaîne perfluorée.

Les résultats sont récapitulés dans le tableau I. L'iodhydrine en $C_4F_9$ se présente sous forme d'un liquide de point d'ébullition 70–75° C sous 2 Torr, celle en $C_8F_{17}$ sous forme d'un solide blanc sublimable de point de fusion 82° C.

Tableau I

| Exemples | 2 | 3 | 4 | 5 |
|---|---|---|---|---|
| [alcool allylique] / [$R_FI$] mole/mole | 5 | 5 | 5 | 5 |
| [$R_FI$] / [$Hg_2I_2$] (mole/mole) | 13,3 | 26,6 | 9 | 13,3 |
| [$Hg_2I_2$] / [$HgI_2$] (mole/mole) | 0,25 | 0,125 | 0,5 | 0,25 |
| DMF cc/mole $R_FI$ | 250 | 250 | 250 | 250 |
| $R_F$ | $C_4F_9$ | $C_4F_9$ | $C_4F_9$ | $C_8F_{17}$ |
| T °C | 35 | 35 | 35 | 35 |
| Transformation du $R_FI$ en produits d'addition | 100 % | 100 % | 100 % | 100 % |
| Temps total de réaction en h | 1,4 | 1,5 | 2 | 1,5 |

Exemples 6 à 11

On répète l'exemple 1 mais en opérant avec divers alcools. Les résultats sont donnés dans le tableau II ci-dessous.

Tableau II

| Ex. | Alcool | Durée | Nbre d'alcools isomères géométriques | Taux de transformation du $R_FI$ en produit d'addition |
|---|---|---|---|---|
| 6 | Butène-1 ol-4 | 4 h | 1 | 100 % |
| 7 | Butène-4 ol-1 | ~4 j | 2 | 100 % |
| 8 | Butène-2 ol-1 | 4 h 30 | 3 | 100 % |
| 9 | Hexène-3 ol-1 | 7 j | 4 | 95 % |
| 10 | Butène-3 ol-2 | 2 h | 2 | 99 % |
| 11 | Méthyl allyl alcool | 4 j | 2 | 95 % |

Exemple 12

Dans les conditions expérimentales de l'exemple 2 mais en opérant à 50° C et en remplaçant les sels de mercure par des quantités équivalentes d'acétylacétonates de $Mn^{3+}$ et $Mn^{2+}$, après une durée de réaction de 96 heures la transformation de $C_4F_9I$ en (perfluorobutyl)-3 iodo-2 propanol ($R_F-CH_2-CHI-CH_2OH$) est totale.

### Revendications

1. Procédé de préparation d'iodo-alcools à chaîne perfluoroalkyle par réaction d'iodo-perfluoroalcanes sur des alcools éthyléniques caractérisé en ce que la réaction est conduite à une température comprise entre 35 et 50° C en présence d'un catalyseur constitué soit par le couple iodure mercureux/iodure mercurique, soit par le couple acétate mercureux/iodure mercurique, soit par le couple acétylacétonate manganeux/acétylacétonate manganique.

2. Procédé selon la revendication 1, caractérisé en ce que l'alcool éthylénique est utilisé en excès par rapport à l'iodo-perfluoroalcane.

3. Procédé selon l'une ou l'autre des revendications 1 et 2, caractérisé en ce que dans le catalyseur le rapport molaire du sel de métal à valence inférieure au sel de métal à valence supérieure est inférieur à 1.

4. Procédé selon l'une quelconque des revendications 1 à 3, caractérisé en ce que l'alcool éthylénique est l'alcool allylique.

### Patentansprüche

1. Verfahren zur Herstellung von Jodalkoholen mit Perfluoralkylkette durch Umsetzung von Jodperfluoralkanen mit äthylenisch ungesättigten Alkoholen, dadurch gekennzeichnet, daß die Reaktion bei einer Temperatur zwischen 35 und 50° C in Gegenwart eines Katalysators durchgeführt, der entweder aus dem Paar Quecksilber-I-jodid/Quecksilber-II-jodid oder dem Paar Quecksilber-I-acetat/Quecksilber-II-jodid oder dem Paar Mangan-II-acetylacetonat/Mangan-III-acetylacetonat besteht.

2. Verfahren nach Anspruch 1, dadurch gekennzeichnet, daß der äthylenisch ungesättigte Alkohol, bezogen auf das Jodperfluoralkan im Überschuß angewendet wird.

3. Verfahren nach Anspruch 1 oder 2, dadurch gekennzeichnet, daß das Molverhältnis des Metallsalzes der geringeren Wertigkeit zum Metallsalz der höheren Wertigkeit in dem Katalysator kleiner als 1 ist.

4. Verfahren nach irgendeinem der Ansprüche 1 bis 3, dadurch gekennzeichnet, daß der äthylenisch ungesättigte Alkohol Allylalkohol ist.

### Claims

1. Process for the preparation of iodo-alcohols with a perfluoroalkyl chain by reacting iodo-perfluoroalkanes with ethylenic alcohols, characterised in that the reaction is carried out at a

temperature of between 35 and 50° C in the presence of a catalyst consisting either of the combination of mercurous iodide and mercuric iodide or of the combination of mercurous acetate and mercuric acetate or of the combination of manganous acetylacetonate and manganic acetylacetonate.

2. Process as claimed in claim 1, characterised in that the ethylenic alcohol is used in an excess relative to the iodo-perfluoroalkane.

3. Process as claimed in either of claims 1 and 2, characterised in that, in the catalyst, the molar ratio of the metal salt having a lower valency to the metal salt with a higher valency is less than 1.

4. Process as claimed in any of claims 1 to 3, characterised in that the ethylenic alcohol is allyl alcohol.